# EUROPÄISCHE PATENTSCHRIFT

(11) **EP 1 165 881 B1**
(45) Veröffentlichungstag und Bekanntmachung des Hinweises auf die Patenterteilung: **11.09.2002**
(21) Anmeldenummer: 00907576.3
(22) Anmeldetag: 17.02.2000
(51) Int. Cl.: D21C 9/147, D21C 9/10, D21C 5/00, C12N 9/02, C11D 3/386

(54) **MEHRKOMPONENTENSYSTEM ZUR ENZYMKATALYSIERTEN OXIDATION VON SUBSTRATEN SOWIE VERFAHREN ZUR ENZYMKATALYSIERTEN OXIDATION**
MULTI-COMPONENT SYSTEM FOR THE ENZYME-CATALYSED OXIDATION OF SUBSTRATES AND METHOD FOR CARRYING OUT ENZYME-CATALYSED OXIDATION
SYSTEME MULTICOMPOSANT POUR L'OXYDATION DE SUBSTRATS CATALYSEE PAR DES ENZYMES ET PROCEDE D'OXYDATION CATALYSEE PAR DES ENZYMES

(30) Priorität: 04.03.1999 DE 19909546
(43) Veröffentlichungstag der Anmeldung: 02.01.2002
(73) Patentinhaber: Consortium für elektrochemische Industrie GmbH, 81379 München (DE)
(72) Erfinder: CANDUSSIO, Anton, D-81825 München (DE)
(74) Vertreter: Potten, Holger
(86) Internationale Anmeldenummer: EP0001290
(87) Internationale Veröffentlichungsnummer: WO00052257

(56) Entgegenhaltungen:
- WO-A-92/09741
- US-A- 5 691 193
- OKAZAKI, M. ET AL.: "Partial purification and characterization of Manganese- oxidizing factors of Pseudomonas fluorescens GB-1" APPLIED AND ENVIRONMENTAL MICROBIOLOGY, Bd. 63, Nr. 12, Dezember 1997 (1997-12), Seiten 4793-4799, XP000913537

## Beschreibung

Die vorliegende Erfindung betrifft ein Mehrkomponentensystem zur mediatorabhängigen enzymkatalysierten Oxidation von Substraten sowie Verfahren zur enzymkatalysierten Oxidation.

Mehrkomponentensysteme zur mediatorabhängigen enzymkatalysierten Oxidation von Substraten sowie entsprechende Verfahren sind prinzipiell bekannt. Mediatoren sind in diesem Zusammenhang generell solche Verbindungen, die von Oxidoreduktasen oxidiert werden können, also zunächst ebenfalls Substrate von enzymatischen Oxidationskatalysatoren. Besonders ausgezeichnet sind Mediatoren nun dadurch, daß die oxidierte Form des Mediators (der aktivierte Mediator, zum Beispiel ein Mediatorradikal oder ein Mediatorkation) eine ausreichend lange Lebensdauer hat, um von der Oxidoreduktase zum eigentlichen Substrat des Oxidationssystems zu diffundieren und mit diesem zu interagieren. Bei der Interaktion zwischen aktiviertem Mediator und Substrat wird das Substrat vom Mediator oxidiert. Durch die Oxidation des Substrates kann der Mediator entweder regeneriert (katalytisches Oxidationssystem) oder inaktiviert (stöchiometrisches Oxidationssystem) werden. Wird der Mediator regeneriert, steht er für einen neuen Katalysezyklus zur Verfügung. Die vom Substrat durch den Mediator auf die Oxidoreduktase übertragenen Elektronen werden wie bei direkten enzymatischen Oxidationsverfahren auf terminale Elektronenakzeptoren wie Sauerstoff oder Peroxid übertragen.

Bei Verfahren bei denen Peroxid (direkt oder aus Vorstufen freigesetzt) als terminaler Elektronenakzeptor dient, können nur Peroxidasen als Oxidoreduktasen verwendet werden. Eine Reihe solcher Verfahren sind zwar bekannt, sie sind jedoch mit zahlreichen Nachteilen behaftet:
Peroxidasen sind in der Herstellung zu teuer um eine Verwendung in technischen Prozessen wie der Papierbleiche oder in Waschmitteln zu finden. Ferner ist die Zudosierung von Peroxidasen problematisch, denn in den für die Wirksamkeit des Verfahrens notwendigen Konzentrationen wirkt Peroxid inaktivierend auf die Peroxidase. Viele der als Mediatoren für Peroxidasen beschriebene Verbindungen sind technisch nicht einsetzbar, weil sie stark gefärbt sind, toxikologisch oder ökotoxikologisch bedenklich sind, nicht oder nur schlecht bioabbaubar sind und nicht in ausreichenden Mengen oder billig genug verfügbar sind. Verfahren bei denen Peroxid als terminaler Elektronenakzeptor dient, sind daher für einen wirtschaftlich sinnvollen technischen Einsatz bisher nicht geeignet.

Bei Verfahren, bei denen Sauerstoff als terminaler Elektronenakzeptor für die aus dem zu oxidierenden Substrat stammenden Elektronen dient, werden Oxidasen als Oxidoreduktasen verwendet. Es sind zwei Gruppen von Oxidasen beschrieben, die in mediatorabhängigen enzymatischen Oxidationsverfahren eingesetzt werden können: Laccasen (Boubonnais & Paice (1990) FEBS LETTERS Vol 267 (1), p. 99 - 102, WO 94/29510) und Tyrosinasen (WO 94/29510). Anders als bei Peroxidasen, bei denen eine prosthetische Hämgruppe als redoxaktives Zentrum wirkt, wird der Elektronentransfer bei Laccasen und Tyrosinasen durch vier in vier entsprechenden Kupferbindedomänen dieser Oxidasen koordinierte Kupferionen ('blue copper'-Oxidasen) katalysiert. Dabei werden nacheinander insgesamt vier Elektronen von Mediatoren aufgenommen, die dann in einem Vierelektronentransfer auf molekularen Sauerstoff übertragen werden. Der Sauerstoff wird dabei zu Wasser reduziert, die Oxidase wird für einen neuen Reaktionszyklus regeneriert. Eine Reihe von Mediatoren, die den Elektronentransfer vom zu oxidierenden Substrat auf die Oxidasen katalysieren, sind beispielsweise in WO 94/29510, WO 97/06244, WO 96/10079 oder WO 95/01426 beschrieben.

Es ist bekannt, diese mediatorabhängigen enzymatischen Oxidationsverfahren mit Oxidasen und Sauerstoff zur Kohleverflüssigung (WO 94/29510), zur Zellstoffbleiche (WO 94/29510), zur organischen Synthese (Potthast et al. (1995) J. Org. Chem., Vol. 60, p. 4320 - 4321) als Bleichsystem in Waschmitteln und Geschirrspülmitteln (WO 97/06244), zur Jeansbleiche (WO 96/12846), zur Verhinderung eines Farbtransfers bei der Wäsche (WO 98/23716) oder zum Abbau polyzyklischer aromatischer Kohlenwasserstoffe (Johannes et al. (1996) Appl. Microbiol. Biotechnol., Vol. 46, p. 313 - 317) zu verwenden.

Da jedoch die Mediatoren Eigenschaften aufweisen, die einem technischen Einsatz entgegenstehen, haben auch die enzymatischen Oxidationsverfahren, die Oxidasen verwenden, gravierende Nachteile:
- Das in WO 94/29510 als besonders wirksamer Mediator beschriebene HBT ist nicht biologisch abbaubar (Amann (1997) 9^{th} Int. Symp. on Wood and Pulp. Chem., F4-1 - F4-5).
- Viele Mediatoren wie HBT und Violursäure inaktivieren in ihrer aktiven Form die verwendeten Oxidasen (Amann (1997) 9^{th} Int. Symp. on Wood and Pulp. Chem., F4-1 - F4-5) und benötigen daher einen hohen Enzymeinsatz im jeweiligen Verfahren.
- Eine Gruppe sehr aktiver Mediatoren ist gekennzeichnet durch eine N-O- Gruppierung und enthält damit mind. ein N-Atom pro Mediatormoleküle (WO 94/29510). Daraus ergeben sich die beim Einsatz N-O - haltiger Verbindungen bekannten Probleme bezüglich Toxizität und Bioabbaubarkeit. Biologische Abwasserreinigungssysteme in der Zellstoffindustrie können zudem häufig keine zusätzlichen N-Frachten verarbeiten.
- Einige der Mediatoren wie z. B. ABTS bilden stark gefärbte Radikale und führen daher zu einer ungewollten Verfärbung des zu oxidierenden Substrates.
- Die meisten der Mediatoren enthalten N- oder S-Atome und sind daher in der Herstellung relativ teuer.

Diese Nachteile limitieren bisher auch einen breiten technischen Einsatz solcher Systeme zur Oxidation von Substraten.

Von Archibald & Roy (Archibald & Roy (1992) Appl. Environ. Microbiol. Vol. 58, p. 1496 - 1499) wurde ein Verfahren beschrieben, bei dem eine Redoxkaskade bestehend aus Phenolen und Mn²⁺/Mn³⁺ als Mediator zwischen Oxidase und dem zu oxidierenden Substrat wirkt. Als terminalen Elektronenakzeptor verwendet das System Sauerstoff. Als Oxidase wird eine Laccase verwendet. Als primäres Substrat werden von der Laccase zunächst Phenole wie m-Hydroxybenzoesäure oder m-Cresol oxidiert. In Gegenwart von Komplexbildnern wie Pyrophosphat-Ionen kann dann sekundär unter Beteiligung der oxidierten Phenole Mn²⁺ zu Mn³⁺ oxidiert werden. Für dies System konnte nicht gezeigt werden, daß auch nicht-phenolische Substrate oxidiert werden können. Auch konnte mit einem System, bei dem nur Laccase, Mn²⁺ und ein Komplexbildner zur Ligninoxidation eingesetzt wurde, eine Delignifizierung nicht nachgewiesen werden. Dies mediatorabhängige enzymatische Oxidationssystem stellt technisch keine wesentliche Verbesserung gegenüber den anderen genannten Systemen dar, weil es an Stelle der Mediatoren nun eine Kombination aus Phenolen, Komplexbildnern und Mn-Ionen verwendet und die obligate Verwendung von phenolischen Mediatoren aus toxikologischen Gründen dem Einsatz des Verfahrens im Wege steht. Ferner verhindern auch die langen Reaktionszeiten von > 15 h einen praktischen Einsatz dieses Systems.

Aufgabe der vorliegenden Erfindung war es ein Mehrkomponentensystem für eine mediatorabhängige enzymatische Oxidation von Substraten bereitzustellen, welche die Nachteile der bekannten Mehrkomponentensysteme nicht aufweist und daher eine einfache und kostengünstige Umsetzung des zu oxidierenden Substrats ermöglicht.

Die Aufgabe wird gelöst durch ein Mehrkomponentensystem umfassend einen Oxidationskatalysator, ein Oxidationsmittel, und einen Mediator, dadurch gekennzeichnet, daß
a) der Oxidationskatalysator ausgewählt ist aus der Gruppe der Manganoxidasen,
b) das Oxidationsmittel ausgewählt ist aus der Gruppe Sauerstoff und sauerstoffhaltige Verbindungen,
c) der Mediator ausgewählt ist aus der Gruppe der Verbindungen, die Mn-Ionen enthalten.

Vorzugsweise umfaßt das erfindungsgemäße Mehrkomponentensystem einen Komplexbildner, der ausgewählt ist aus der Gruppe der Komplexbildner, die Mn-Ionen komplexieren können.

Im Sinne der vorliegenden Erfindung sind unter Manganoxidasen solche Oxidasen zu verstehen, die Mn oder Mn-Ionen direkt oxidieren und die dabei gewonnen en Elektronen auf Sauerstoff übertragen. Vorzugsweise handelt es sich um solche Oxidasen, die in Gegenwart von Sauerstoff und Komplexbildnern in der Lage sind, Mn²⁺ zu Mn³⁺ zu oxidieren. Bevorzugt sind Manganoxidasen, die als redoxaktive katalytische Gruppe Kupferionen enthalten.

Manganoxidasen können beispielsweise aus bekannten Mikroorganismen gewonnen werden. Dazu werden solche Mikroorganismen unter Bedingungen, unter denen sie Manganoxidasen bilden, angezogen. In der erfindungsgemäßen Zusammensetzung können als Manganoxidasen im einfachsten Fall mechanisch, enzymatisch oder chemisch aufgeschlossene Zellpräparationen der kompletten manganoxidase-haltigen Mikroorganismen eingesetzt werden. Es ist jedoch ebenso möglich, manganoxidase-haltige Kulturüberstände oder isolierte Manganoxidasen einzusetzen.

Manganoxidasen werden zum Beispiel gebildet von Leptothrix discophora, Bacillus SG-1 und Pseudomonas sp. (Nealson et al. (1989) Beveridge & Doyle (eds.) Metal ions and bacteria, John Wiley and sons, Inc., New York, p: 383-411). Die für die Manganoxidasen aus Leptothrix discophora und Bacillus SG-1 kodierenden Gene sind kloniert und sequenziert (Corstjens et al. (1997) Geomicrobiol. Journal, Vol. 14, p. 91-108 und van Waasbergen et al. (1996) Journal of Bacteriology, Vol. 178 (12), p. 3517 - 3530). Beide Gene kodieren für Proteine, die die aus 'blue copper' - Oxidasen bekannten, kupfer-bindenden Sequenzmotive enthalten und deren Manganoxidase - Aktivität durch Kupferzugabe erhöht wird.

Neben den genannten Manganoxidase - produzierenden Mikroorganismen können auch andere Mikroorganismen als Quelle für Manganoxidasen verwendet werden. So können z. B. bei Mikroorganismen bei denen die Manganoxidase in die Spore eingelagert wird wie z. B. Bacillus SG-1, auch die isolierten Sporen als Enzymquelle im erfindungsgemäßen Verfahren eingesetzt werden.

Es können ferner Manganoxidasen verwendet werden, die mit rekombinanten Produktionsverfahren erzeugt wurden. Unter rekombinanten Produktionsverfahren sind dabei alle Verfahren zu verstehen, bei denen die für Manganoxidasen kodierenden Gene aus den natürlichen Produzenten isoliert werden und dann mit bekannten Methoden in geeignete Produktionsstämme, bei denen es sich auch um die ursprünglichen Enzymproduzenten handeln kann, eingebracht wurden.

Als Mediator dienen in der erfindungsgemäßen Zusammensetzung Manganionen. Diese können in beliebiger Oxidationsstufe in den Prozeß eingesetzt werden.

Vorzugsweise werden Manganionen der Oxidationsstufe +2 oder +3 eingesetzt.

Mangan der Oxidationsstufe +2 wird bevorzugt in Form von Mangansulfat oder Manganchlorid eingesetzt.

Mangan der Oxidationsstufe +3 wird bevorzugt in Form löslicher Mangankomplexe eingesetzt. Beispiele für solche Mangankomplexe sind Mangan/Formiat, Mangan/Laktat, Mangan/Oxalat oder Mangan/Malonat.

Der Mediator (z.B. das Mn³⁺ Ion) nimmt ein Elektron von der zu oxidierenden Substanz auf. Die Substanz wird dadurch oxidiert, und das Mn Ion selbst reduziert (z.B. zu Mn²⁺ Ion). In dieser Form transportiert das Mn-Ion das aufgenommene Elektron zur Manganoxidase, gibt es dort ab und oxidiert wieder zur anfänglichen Oxidationsstufe (z.B. zum Mn³⁺ Ion). Der Manganoxidase dient Sauerstoff als Oxidationsmittel und damit als terminaler Elektronenakzeptor.

Der Sauerstoff kann mittels bekannter chemischer oder enzymatischer sauerstoffgenerierender Systeme direkt in der Reaktion erzeugt werden. Er kann ebenso durch elektrische Hydrolyse von Wasser erzeugt werden, oder er kann direkt gasförmig oder flüssig eingesetzt werden.

Bevorzugt wird Sauerstoff entweder direkt gasförmig oder in Form von flüssigem Sauerstoff oder als sauerstoffhaltiges Gasgemisch wie zum Beispiel Luft eingesetzt. Besonders bevorzugt wird Sauerstoff als sauerstoffhaltiges Gasgemisch wie zum Beispiel Luft eingebracht.

Vorzugsweise wirkt das von Manganoxidasen durch direkte Oxidation von Mn²⁺ gebildete Mn³⁺ in Gegenwart eines Komplexbildners.

Als Komplexbildner sind vorzugsweise solche Verbindungen geeignet, die Mn³⁺ komplexieren und dadurch stabilisieren.

Bevorzugt werden Komplexbildner eingesetzt, die keinen Stickstoff enthalten, leicht bioabbaubar und toxikologisch unbedenklich sind. Solche Komplexbildner sind beispielsweise Formiat, Laktat, Malonat oder Oxalat. Okazaki, u. et al.: 'Partial purification and characterization of Manganeze-oxidizing factors of Pseudomones fluorescens GB-1 Applied and Environmental Microbiology, Bd. 63, Nr. 12, December 1997 (1997-12), Seiten 4793-4799, offenbart ein Mn²⁺ oxidierendes System aus Pseudomones fluorescens 66-1.

Das erfindungsgemäße System weist folgende Vorteile gegenüber bekannten Systemen auf:
- Die Problematik der Inaktivierung der Oxidoreduktasen wie sie z.B. bei Verwendung von Peroxid als Elektronenakzeptor vorkommt tritt bei der erfindungsgemäßen Zusammensetzung nicht auf, da Sauerstoff als Oxidationsmittel und damit als terminaler Elektronenakzeptor dient.
- Sauerstoff kann technisch einfach und in ausreichenden Mengen dosiert werden.
- Manganoxidasen benötigen für ihre Aktivität im Gegensatz zu Peroxidasen und auch Mangan-Peroxidasen bekanntermaßen keine prosthetischen Gruppen wie z.B. Häm-Gruppen. Damit sind Manganoxidasen in üblichen Produktionssystemen in technischem Maßstab problemlos herzustellen.
- Das erfindungsgemäße System benötigt keine N- oder S-haltigen oder farbige oder schlecht bioabbaubare oder toxisch bedenkliche Mediatoren. Als redoxaktiver Mediator dienen nur Mangan Ionen, die zwischen zwei Oxidationsstufen, vorzugsweise den Oxidationsstufen ²⁺ und ³⁺ wechseln. Eine Inaktivierung dieses Mangan-Mediators kann im Gegensatz zur Inaktivierung von bekannten Mediatoren z.B. durch eine chemische Veränderung nicht stattfinden.

Die erfindungsgemäße Zusammensetzung läßt sich zur Oxidation unterschiedlichster Substrate einsetzen. Bevorzugt lassen sie sich zur Oxidation solcher Substrate einsetzen, die mittels Mn Ionen oxidiert werden können. Besonders bevorzugt lassen sie sich zur Oxidation solcher Substrate einsetzen, die mittels Mn³⁺ Ionen oxidiert werden können

Das erfindungsgemäße Mehrkomponentensystem eignet sich beispielsweise als Bleichsystem in Waschmitteln und Geschirrspülmitteln, zur Zellstoffbleiche, zur Jeansbleiche, zur Behandlung von Abwasser, zur organischen Synthese, zur Verhinderung eines Farbtransfers bei der Wäsche oder zum Abbau polyzyklischer aromatischer Kohlenwasserstoffe. Eine weitere Einsatzmöglichkeit ist beispielsweise die Kohleverflüssigung.

Entsprechende Verfahren sind im Stand der Technik für mediatorabhängige enzymatische Oxidationsverfahren mit Oxidasen und Sauerstoff beschrieben.

Es ist dem Fachmann ein leichtes, die jeweils bekannten Verfahren unter Nutzung der in der vorliegenden Anmeldung genannten Komponenten und Verfahresbedingungen zu modifizieren um die erfindungsgemäße Zusammensetzung für die genannten Zwecke zu verwenden.

Die vorliegende Erfindung betrifft ferner ein Verfahren zur Oxidation eines Substrats dadurch gekennzeichent, daß eine Manganoxidase in Anwesenheit von Sauerstoff und ggf. eines Komplexbildners für Mn-Ionen durch direkte Mn²⁺ Oxidation Mn³⁺ - bildet, das Mn³⁺ Ion das Substrat oxidiert, dabei selbst zu Mn²⁺ reduziert wird und wiederum zur direkten Oxidation durch die Manganoxidase zur Verfügung steht.

Im erfindungsgemäßen Verfahren wird das Substrat vorzugsweise in Form einer wässrigen Lösung, Mischung oder Suspension eingesetzt.

Im erfindungsgemäßen Verfahren werden vorzugsweise zwischen 0,001 und 50 Milligramm aktive Manganoxidase pro Liter Reaktionsvolumen eingesetzt.

Die Manganoxidase wird vorzugsweise in granulärer Form, als Lösung, als Suspension oder mit einem Trägermaterial in den Reaktionsansatz eingebracht.

Besonders bevorzugt wird die Manganoxidase in Form einer Suspension, die zwischen 0,5 und 50 Gewichtsprozent Enzym in einem nichtionischen Detergenz enthält, in den Reaktionsansatz eingebracht.

Im erfindungsgemäßen Verfahren wird vorzugsweise Mangan der Oxidationsstufen Mn²⁺ oder Mn³⁺ als Redoxmediator verwendet. Manganionen der Oxidationsstufe 2+ oder 3+ können direkt in dieser Oxidationsstufe eingesetzt werden oder aus Manganionen anderer Oxidationsstufen wie Mn4+ oder Mn7+ im Prozeß erzeugt werden.

Mangan der Oxidationsstufe +3 wird bevorzugt in Form löslicher Mangankomplexe wie Mangan/Formiat, Mangan/Laktat, Mangan/Oxalat oder Mangan/Malonat dem Reaktionsansatz zugesetzt.

Bevorzugt wird im erfindungsgemäßen Verfahren Mangan der Oxidationsstufe +2 eingesetzt. Besonders bevorzugt wird Mangansulfat oder Manganchlorid verwendet.

Im erfindungsgemäßen Verfahren werden Manganionen in Konzentrationen zwischen 0,005 mM und 50 mM verwendet. Bevorzugt werden Manganionen in Konzentrationen zwischen 0,05 mM und 5 mM und besonders bevorzugt in Konzentrationen zwischen 0,1 mM und 1 mM verwendet.

Je nach konkreter Anwendung kann das benötigte Mangan bereits im zu oxidierenden Substrat enthalten sein. Ein Beispiel für ein erfindungsgemäßes Verfahren, bei dem in der Regel keine externen Manganionen zugesetzt werden müssen, ist die Zellstoffbleiche. Holz und der daraus gewonnene Zellstoff enthält häufig bereits natürlicherweise eine für das erfindungsgemäße Oxidationsverfahren ausreichend hohe Menge an Manganionen.

Im erfindungsgemäßen Verfahren wird Sauerstoff vorzugsweise mit einem Partialdruck von 0,05 - 5 bar eingesetzt. Besonders bevorzugt wird Sauerstoff mit einem Partialdruck von 0,1 bis 2,5 bar eingesetzt. Insbesondere bevorzugt wird Sauerstoff mit einem Partialdruck von 0,2 bis 1 bar eingesetzt.

Im erfindungsgemäßen Verfahren werden die genannten Komplexbildner vorzugsweise in Konzentrationen zwischen 1 mM und 500 mM verwendet. Bevorzugt werden geeignete Komplexbildner in Konzentrationen zwischen 5 mM und 100 mM und besonders bevorzugt in Konzentrationen zwischen 10 mM und 50 mM verwendet.

Das erfindungsgemäße Oxidationsverfahren kann zur Oxidation von allen Verbindungen verwendet werden, die durch Mn³⁺ - Ionen oxidierbar sind. Erfindungsgemäße Verfahren können zum Beispiel verwendet werden zur Ligninoxidation bei der Papier und Zellstoffherstellung, als Oxidationssystem in Waschmitteln und Geschirrspülmitteln, zur enzymatischen Farbstoffbleiche, zur enzymatischen Textilbleiche, als System zur Verhinderung des Farbtransfers bei der Wäsche, zur spezifischen Oxidation organischer Zielmoleküle in der organischen Synthese, zur oxidativen Abwasserbehandlung, zum Abbau chlorierter Kohlenwasserstoffe, zur Spaltung polyzyklischer aromatischer Kohlenwasserstoffe und zur Verflüssigung von Kohle.

Die folgenden Beispiele dienen der weiteren Erläuterung der Erfindung.

### Beispiel 1:

### Isolation von Manganoxidase - produzierenden Mikroorganismen

### 1.a. Isolierung Mangan oxidierender Mikroorganismen:

Mangan oxidierende Mikroorganismen, besonders Pilze und Bakterien, wurden auf Indikatorplatten nach Krumbein & Altmann (Krumbein & Altmann (1973) Helgoländer wiss. Meeresunters., Vol. 25, p. 347 - 356) isoliert.

In Gegenwart von Mangan-Ionen der Oxidationsstufen Mn3+ - Mn7+ bildet Berbelinblau bei pH 4 - 7 einen intensiv blau gefärbten Farbstoff. Bei einer erhöhten Konzentration an Berbelinblau entsteht in Anwesenheit geeigneter Mn-Ionen das blaue Oxidationsprodukt bis pH 10.

Für das Plattenscreening wurden Nährmedien mit farblosem Berbelinblau (N, N'-Dimethylamino-p,p'-triphenylmethan-o''sulfonsäure) versetzt. Bei Konzentrationen von 10⁻⁴ - 10⁻⁵ g Berbelinblau pro 100 ml Nährmedium fand noch keine Hemmung des bakteriellen Wachstums statt.

Zur Isolation von heterotrophen manganoxidierenden Bakterien wurde folgendes Medium verwendet:
3,5 g/l Difco-Bacto-Pepton
0,8 g/l MnSO₄ x H₂O
100 mg/l FeSO₄ x 7 H₂O
15 g/l Difco-Bacto-Agar
750 ml Meerwasser
245 ml destilliertes Wasser
10 ml Berbelinblau - Stammlösung (4 g/l)
pH 7,6

Auf solchen Indikatorplatten wurden bakterienhaltige Proben wie Meerwasserproben, Sedimentproben, Bodenproben, Proben aus Erzhalden usw. in geeigneter Verdünnung oder Konzentration so aufgebracht, daß pro Indikatorplatte zwischen 100 und 300 Einzelkolonien gewachsen sind.

Bakterien, die nach 5 bis 10 Tagen Inkubationszeit auf solchen Indikatorplatten blaue Kolonien (d.h. die manganoxidierende Aktivität war zellassoziiert) bildeten oder um die Kolonien blaue Höfen aufwiesen (d.h. Mangan oxidierende Enzyme wurden in den Kulturüberstand sekretiert), wurden weiter analysiert.

### 1.b. Nachweis von Manganoxidasen in Berbelinblau-oxidierenden Mikroorganismen

Mikroorganismen, die wie in la beschrieben isoliert wurden, können Manganoxidasen produzieren, die Blaufärbung könnte aber auch durch Manganperoxidasen oder durch Oxidation von Fe²⁺ hervorgerufen worden sein. Der spezifische Nachweis der Manganoxidase - Aktivität wurde, wie von Boogerd & de Vrind (Boogerd & de Vrind (1987) J. Bacteriol., Vol. 169 (2), p. 489 - 494) beschrieben, geführt:

Zunächst wurden die zu untersuchenden Mikroorganismen jeweils in 1 1 des zur Isolation verwendeten Mediums ohne Agar und Berbelinblau - Zusatz für 10 Tage angezogen. Anschließend wurden die Zellen durch Zentrifugation (15 min, 10000 x g, 4°C) entfernt. Bei Zellen, die auf den in Beispiel la beschriebenen Indikatorplatten blaue Kolonien bildeten, wurde das Zellpellet für die im folgenden beschriebene Gelelektrophorese verwendet. Bei Zellen, die auf Indikatorplatten blaue Höfe bildeten, wurde der zellfreie Kulturüberstand durch Ultrafiltration an einer UF-Membran mit einem Retentionsvermögen für Partikel > 10.000 D auf ein fünfzigstel seines Ausgangsvolumens aufkonzentriert. Das Konzentrat oder das Zellpellet wurde mit dem gleichen Volumen eines Puffers (0,125 M Tris/Cl pH 6,8, 20 % Glyzerin, 2% SDS, 10 % 2-Mercaptoethanol, 0,01 % Bromphenolblau) vermischt. Jeweils 50 µl einer solchen Mischung wurden auf einem 10% Polyacrylamidgel elektrophoretisch aufgetrennt. Nach der Elektrophorese wurde das Gel vier mal für 15 min mit deionisiertem Wasser gewaschen. Anschließend wurde das Gel für 2 h in 100µM MnCl₂ in 10 mM Hepes ph 7,5 inkubiert. In Proben, die Manganoxidasen enthielten, bildeten sich bei dieser Prozedur an der Stelle, an der nach der Elektrophorese die Manganoxidasen im Gel lokalisiert waren, ein brauner Niederschlag aus Manganoxid. Die so beobachtete Manganoxid-Bildung wurde jeweils von direkt Mn²⁺ - oxidierenden Manganoxidasen verursacht, weil kein Peroxid vorhanden war und keine anderen Ionen als Mn²⁺ im Ansatz vorlagen.

### Beispiel 2:

### Produktion und Isolation von Manganoxidasen

### 2.a. Manganoxidase - enthaltende Sporen aus Bacillus

Nach der in Beispiel 1 beschriebenen Methode konnten Bacillus - Isolate isoliert werden, die in ihren Sporen Manganoxidase eingelagert haben. Für technische Anwendungen wurden solche Manganoxidase enthaltenden Sporen direkt eingesetzt oder es wurden die manganoxidase-enthaltenden Sporenhüllen präpariert und verwendet. Solche Sporen oder Sporenhüllen wurden wie folgt gewonnen:

### Anzucht:

Für die Gewinnung von manganoxidase-haltigen Sporen wurde ein geeigneter Bacillus Stamm aerob bei 25°C in folgendem Medium angezogen:

2 g/l Pepton (Difco), 0,5 g/l Hefeextrakt (Difco), 10 µg/l Eisen-EDTA, 100 µg/l sterilfiltriertes MnCl₂ x 4 H₂O in 50 mM Tris in 80% natürlichem Meerwasser, pH 7,0.
Nach einer Anzuchtszeit von 10 Tagen waren > 95% aller Zellen sporuliert.

### Sporenpräparation:

Die Sporen wurden durch Zentrifugation (30 min 10000 x g, 4°C) geerntet, mit deionisiertem Wasser gewaschen und in 10 mM Tris/Cl pH 7,0 suspendiert (0,1 g/ml). Für eine Präparation der Sporenhülle wurde dieses Material wie unten beschrieben weiterverarbeitet. Wurden im erfindungsgemäßen Verfahren die Sporen direkt verwendet, wurden diese wie folgt weiterbehandelt:

Zur Suspension wurden 50 µg/ml Lysozym gegeben, der Ansatz wurde für 30 min bei 37°C inkubiert, anschließend wurden die Sporen mit 1M NaCl, 0,15 M NaCl, 0,1 % SDS und fünfmal mit deionisiertem Wasser gewaschen. So gereinigte Sporen wurden bis zur Verwendung im erfindungsgemäßen Verfahren in deionisiertem Wasser bei 4°C gelagert.

### Präparation der Sporenhüllen:

Die gesamte Prozedur wurde, wenn nicht anders angegeben, bei Raumtemperatur durchgeführt. Mit Ausnahme der 1% SDS-Lösung und des zum Waschen verwendeten deionisierten Wassers enthielten alle Lösungen 10 mM EDTA, pH 7,5 und PMSF (0,3 Gewichtsprozent). Die gereinigten Sporen wurden wie oben beschrieben in 10 mM Tris pH 7,0 suspendiert (0,1 g/ml) und mit einem gleichen Volumen Glasperlen (Durchmesser 10 - 50 µm) versetzt. Anschließend wurde die Suspension für 15 min in 30 sec - Intervallen bei 0°C mit einem Ultraschall - Zelldisruptor (Firma Sonifier) bei maximaler Amplitude aufgeschlossen. Nach der Behandlung wurde die Suspension für 5 min auf Eis inkubiert; der Überstand wurde anschließend von den sich dabei absetzenden Glasperlen abgenommen. Die Glasperlen wurden anschließend zweimal mit einem ihrem Volumen entsprechenden Volumen von 10 mM Tris pH 7,0 gewaschen. Der erste Überstand und die Überstände aus der Waschprozedur wurden vereinigt und für 15 min bei 15.000 g zentrifugiert. Der Überstand wurde anschließend abgenommen, der Niederschlag wird in 10 mM Tris pH 7,5 suspendiert und für 30 min bei 37°C mit Lysozym (100 µg/ml) behandelt. Der Ansatz wurde erneut für 15 min bei 15.000 g zentrifugiert. Der die Sporenhüllen enthaltende Niederschlag wurde gewaschen mit: 1 M NaCl, 0,14 M NaCl, 1 % SDS und 5 mal mit deionisiertem Wasser.

### 2.b. Manganoxidase aus Leptothrix discophora

Nach der in Beispiel 1 beschriebenen Methode können scheidenbildende Organismen wie Leptothrix discophora - Isolate, die eine Manganoxidase produzieren, erhalten werden. Solche Isolate können auch aus kommerziellen Stammsammlungen erworben werden (Leptothrix discophora ATCC 51168, Leptothrix discophora ATCC 51169,). Besonders geeignet zur Produktion von Manganoxidasen sind Derivate von Leptothrix discophora, die die Fähigkeit zur Bildung von Scheiden verloren haben. Solche Derivate entstehen spontan, wenn scheidenbildende Stämme kontinuierlich und für längere Zeit unter Laborbedingungen angezogen werden (Adams & Ghiorse (1986) Arch. Microbiol., Vol. 145, p. 126 - 135). Solche nicht mehr scheidenbildende Derivate sekretieren Manganoxidasen in das Kulturmedium.

Das folgende Beispiel beschreibt die Produktion und Gewinnung von Manganoxidasen in den und aus dem Kulturüberstand von nicht mehr scheidenbildenden Derivaten von Leptothrix discophora, hinterlegt gemäß Budapester Vertrag bei der DSMZ Deutschen

### Sammlung von Mikroorganismen und Zellkulturen GmbH, D-38124 Braunschweig unter der Nummer DSMZ 12667:

### Anzucht:

Leptothrix discophora wurde zur Gewinnung von Manganoxidase in einem Medium angezogen, das folgende Bestandteile enthielt:
0,25 g/l Difco-Pepton
0,25 g/l Difco-Hefeextrakt
0,25 g/l Glukose
0,6 g/l MgSO₄ x 7 H₂O
0,07 g/l CaCl₂ x 2H₂O
0,015 g/l MgSO₄ x H₂O
pro Liter deionisiertem Wasser.
Vor dem Autoklavieren des Mediums wurde der pH-Wert mit 1 M NaOH auf pH 7,6 eingestellt.

Zur Gewinnung von Manganoxidase wurden 45 1 des beschriebenen Mediums mit zwei Litern einer Leptothrix discophora - Vorkultur im selben Medium beimpft. Die Anzucht erfolgte unter Begasung mit Luft (0,2 Volumen Luft pro Volumen Medium und Minute) bei 26°C für 40 Stunden.

### Enzympräparation aus dem Kulturüberstand:

Nach 40 Stunden wurde die Anzucht beendet. Die Zellen von Leptothrix discophora wurden durch Zentrifugation vom Kulturüberstand abgetrennt. Der zellfreie, Manganoxidase enthaltende Kulturüberstand wurde mittels Ultrafiltration an einer UF-Membran mit einem Retentionsvermögen für Partikel > 10.000 D auf 0,5 1 aufkonzentriert. Der aufkonzentrierte Kulturüberstand wurde für die unten geschilderten Beispiele als Manganoxidase-Quelle verwendet.

### Beispiel 3:

### Quantitative Bestimmung der Aktivität von Manganoxidasen mittels N,N,N',N'-Tetramethyl-p-Phenylendiamin (TMPD)

Mn³⁺ - Ionen sind in der Lage, die farblose Verbindung TMPD zu oxidieren. Das Oxidationsprodukt 'Wuster Blau' weist eine intensive blaue Farbe auf, die Zunahme dieser Farbe kann photometrisch bei einer Wellenlänge von 610 nm verfolgt werden.

### Durchführung:

Vor jeder Messung wurde eine TMPD - Stammlösung von 2,1 mM TMPD in destilliertem Wasser frisch zubereitet. Die zu bestimmende Enzympräparation wurde in 10 mM HEPES (N-2-hydroxyethylpiperazin-N'-2-ethansulfonsäure pH7,5) so verdünnt, daß bei der im folgenden beschriebenen Aktivitätsmessung nach 10 min Meßdauer eine OD 610 zwischen 0,5 und 1,5 gemessen wurde.

Als Manganquelle diente eine Lösung von 10 mM MnSO₄ in destilliertem Wasser.

Zur Bestimmung der Manganoxidaseaktivität in Enzymproben wurden parallel jeweils 3 ml der Enzymverdünnungen in HEPES (vgl. oben) mit 0,3 ml TMPD - Stammlösung vermischt. Zu je einem der Parallelansätze wurden zum Zeitpunkt 0 min 10 µl der MnSO₄ - Stammlösung gegeben. Beide Proben wurden gemischt und für 10 min bei 40°C inkubiert. Nach 10 min wurden die Proben kurz zentrifugiert (15 sec; 5000 rpm), anschließend wurde die OD610 in je 1 ml der MnSO₄ - haltigen Überstände bestimmt. Als Referenz diente die Vergleichsprobe ohne MnSO₄. Eine Zunahme der Absorption bei 610 nm um 1,0 entspricht der Bildung von 100 µM 'Wuster Blau'. Eine Einheit (1U) Manganoxidase wurde definiert als die Enzymmenge, die in 1 min zur Bildung von 1 µM 'Wuster Blau' aus TMPD führt.

Für die folgende Messung wurden jeweils konzentrierte, manganoxidase-enthaltende Kulturüberstände aus Beispiel 2 b und 50 µl der Sporenhüllensuspension aus Beispiel 2 a eingesetzt. Folgende Ergebnisse wurden erhalten:

| Enzym aus | OD610 |
|---|---|
| Leptothrix | 1,03 |
| discophora | |
| Bacillus | 0,85 |

### Beispiel 4:

### Oxidation des homogen verfügbaren Substrates Veratrylalkohol in Abhängigkeit von verschiedenen Mangan-Konzentrationen und Komplexbildnern

### Durchführung:

Vom Substrat Veratrylalkohol (3,4-Dimethoxybenzylalkohol (Aldrich)) wurde eine Stammlösung mit 0,25 g / ml in Ethanol hergestellt.

Die Oxidationsreaktion wurde bei 45°C unter leichtem Rühren durchgeführt. Dazu wurden jeweils 22,23 ml einer Lösung mit Komplexbildner (50 mM Formiat, Laktat, Malat oder Oxalat; jeweils pH 7,5) mit MnSO₄ versetzt (0,00, 0,05, 0,50 mM MnSO₄). Dann wurden jeweils 0,268 ml der Veratrylalkohol - Stammlösung zugegeben. Der Ansatz wurde für 10 min bei 45°C äquilibriert, dann wurde die Reaktion durch die Zugabe von 2,5 ml Enzymlösung (Manganoxidase aus Leptothrix discophora, 10 U / ml) gestartet. Nach 8 h wurde die Reaktion mittels HPLC-Analyse auf die Bildung von Veratrylaldehyd hin untersucht. Dazu wurden 0,8 ml Testansatz mit 0,4 ml einer H₂SO₄-Lösung (0,5 mol/l) versetzt. 20 µl dieser Probe wurden auf eine LiChospher 60 RP Select B Trennsäule (Merck 50940) aufgetragen und mit einem H₂O/MeOH-Gemisch (65:35) eluiert. Die Flußrate betrug dabei 1 ml/min. Die Produkte im Eluat wurden mittels UV-Detektor bei 275 nm detektiert.

Die folgende Tabelle zeigt in % die Menge an Veratrylalkohol, die unter den beschriebenen Bedingungen von dem erfindungsgemäßen Verfahren in 8 h zu Veratrylaldehyd oxidiert wurde:

| Komplexbildner | MnSO₄ [mM] | Ohne Mn-Oxidase [% V.aldehyd] | Mit Mn-Oxidase aus L. discophora [% V.aldehyd] |
|---|---|---|---|
| 50 mM Formiat | 0 | 0,00 | 0,00 |
| 50 mMFormiat | 0,05 | 0,01 | 7,24 |
| 50 mM Formiat | 0,5 | 0,00 | 12,46 |
| 50 mM Laktat | 0 | 0,00 | 0,00 |
| 50 mM Laktat | 0,05 | 0,00 | 3,32 |
| 50 mM Laktat | 0,5 | 0,02 | 2,79 |
| 50 mM Malonat | 0 | 0,00 | 0,00 |
| 50 mM Malonat | 0,05 | 0,03 | 14,23 |
| 50 mM Malonat | 0,5 | 0,02 | 22,96 |
| 50 mM Oxalat | 0 | 0,00 | 0,00 |
| 50 mM Oxalat | 0,05 | 0,06 | 17,30 |
| 50 mM Oxalat | 0,5 | 0,07 | 21,44 |

### Beispiel 5:

### Zellstoffbleiche

Mit Sauerstoff delignifizierter Softwood - Kraftzellstoff mit einem Ligningehalt von Kappa 16,5 wurde gewaschen und mit 30 mM Na-Oxalatpuffer (pH 7,5) auf eine Stoffdichte von 5 % gebracht. Anschließend wurde MgSO₄ (0,5 mM) zugegeben. Die Suspension wurde für 60 sec mit einem geeigneten Rührer homogenisiert. Anschließend wurden in zwei Parallelansätzen jeweils 5 U Manganoxidase (Leptothrix, Bacillus) pro g Zellstoff zugegeben. Ein dritter Ansatz ohne Enzymzugabe diente als Kontrolle. Die Ansätze wurden nochmals für 60 sec homogenisiert und in einen temperierbaren, begasbaren Stahlautoklaven eingebracht. Der Autoklav wurde verschlossen, es wird eine Sauerstoffdruck von 3 bar angelegt und der Autoklav wurde für 4 h bei 45°C inkubiert. Anschließend wurde der Reaktionsansatz entleert. Der Zellstoff wurde mit 10 Volumen fließendem 50 °C warmen Wasser gewaschen, anschließend wurden aus dem Zellstoff mit einer 70°C warmen wäßrigen NaOH-Lösung Ligninbruchstücke alkalisch extrahiert. Zur Extraktion wurden pro kg Zellstoff 20 g NaOH verwendet. Nach der Extraktion wurde der Zellstoff erneut mit 10 Volumen fließendem 50 °C warmen Wasser gewaschen. Abschließend wurde der Ligningehalt der Zellstoffproben über den sogenannten Kappa-Wert gemäß der standardisierten Testmethode SCAN-C 1:77 des 'Scandinavian Pulp and Paper Board' bestimmt:

| Enzym | Kappa-Wert | Delignifizierung in % |
|---|---|---|
| Kontrolle ohne Enzym | 15,2 | 7,9 |
| Leptothrix discophora | 13,4 | 18,8 |
| Bacillus Sporenhüllen | 10,7 | 35,1 |

Im Vergleich zur Kontrollprobe konnte bei Verwendung erfindungsgemäßer Oxidationsverfahren eine zusätzliche Delignifizierung zwischen 10,9 und 27,2 % erreicht werden.

### Beispiel 6:

### Unterdrückung von Farbtransfer

Die Versuche zum Nachweis der Unterdrückung von Farbtransfer durch erfindungsgemäße Oxidationssysteme wurden in heizbaren Glasbechern (100 ml Volumen) durchgeführt. Als Kontrolle wurde ein Ansatz verwendet, bei dem 0,25 g eines kommerziellen Waschmittels in 50 ml Wasser gelöst wurden. Die Versuche mit Manganoxidase wurden in 50 ml eines 30 mM Na-Malonatpuffer (pH 7,5) mit 0,5 mM MnSO₄ durchgeführt. Die 50 ml - Ansätze wurden zuerst auf 45 °C erhitzt, dann wurde den Pufferansätzen gegebenenfalls jeweils 0,5 U/ml Manganoxidase zugesetzt. Die Textilmuster aus Baumwolle (jeweils 5 g) wurden den Ansätzen zugegeben, dann wurde zu jedem Ansatz 10 ml einer auf 45°C vorgeheizten Lösung von 15 µM Cibacron Marine C-B (Ciba) als Testfarbstoff zugegeben. Nach einer einstündigen Inkubation bei 45°C wurden die Textilmuster aus den Ansätzen entnommen und mit jeweils 1 1 fließendem 50 °C warmen Wasser gewaschen.

Anschließend wurden die Textilmuster gewaschen und getrocknet. Die Helligkeit der Ausgangsproben und der behandelten Proben wurde anschließend spektroskopisch bestimmt:

In der Tabelle sind die Helligkeiten der behandelten Baumwollmuster im Vergleich zu einer nicht behandelten Probe dargestellt:

| Enzym | Malonatpuffer / MnCl₂ | Wasser/Waschmittel |
|---|---|---|
| kein | 73,3 % | 89 % |
| Leptothrix discophora | 89,2 % | - |
| Bacillus | 92 % | - |

Die in der Tabelle wiedergegebenen Werte zeigen, daß das erfindungsgemäße Oxidationssystem geeignet ist, den Transfer des zugegebenen Farbstoffes auf die Baumwollmuster zu unterbinden. Der Effekt ist in seiner Qualität dem mit kommerziellen Waschmitteln erreichbaren Effekt vergleichbar.

### Beispiel 7:

### Fleckenbleiche (Waschmittelanwendung)

Um die Flecken bleichende Wirkung des erfindungsgemäßen Verfahrens zu zeigen, wurden Waschversuche durchgeführt. Dazu wurden auf weißen Baumwollproben (7,5 cm x 7,5 cm) durch auftropfen von je 0,3 ml einer wäßrigen Lösung von 100 ppm Evan's Blau (erhältlich bei Wako Pure Chemical Industry Co., Osaka) genormte Verunreinigungen erzeugt. Diese Stoffmuster wurden anschließend in heizbaren Glasbechern (100 ml Volumen) unter Rühren (Magnetrührer) erfindungsgemäß mit Manganoxidase, MnSO₄ und geeignetem Komplexbildner in Gegenwart von Sauerstoff behandelt. Als Kontrolle dienten Ansätze, bei denen a) nur Malonatpuffer verwendet wurde, oder b) kein MnSO₄ oder c) keine Manganoxidase zugegeben wurde:

Die Versuche wurden in 50 ml eines 30 mM Na-Malonatpuffer (pH 7,5) mit 0,5 mM MnSO₄, bzw. ohne MnSO₄ durchgeführt. Die 50 ml - Ansätze wurden dabei zuerst auf 45 °C erhitzt, dann wurde bei den Ansätzen, die Manganoxidase enthalten sollen, jeweils 1 U/ml Manganoxidase aus Bacillus bzw. aus Leptothrix zugesetzt. Die verunreinigten Baumwollproben wurden den Ansätzen zugegeben. Nach einer einstündigen Inkubation bei 45°C wurden die Textilmuster aus den Ansätzen entnommen und mit jeweils 1 1 fließendem 50 °C warmen Wasser gewaschen. Anschließend wurden die Textilmuster getrocknet.

Zur Bestimmung der Farbunterschiede der Proben im Vergleich zu der Kontrollprobe ohne Manganoxidase und MnSO₄ wurden die Y, y und x-Werte der luftgetrockneten Baumwollproben mit einem Farbdifferenzmeter (CR-200, Minolta) gemessen. Zur Beurteilung der Bleicheffekte der erfindungsgemäßen Verfahren wurde aus den erhaltenen Werten der Z-Wert (Z=(1-x-y)Y/y) ermittelt.

In der Tabelle sind die Weißen der gemessenen Proben im Vergleich zur Weiße der Kontrolle ohne Enzym und ohne MnSO₄ dargestellt:

| | + MnSO₄ | - MnSO₄ |
|---|---|---|
| Bacillus | 2,7 | -0,3 |
| Leptothrix | 1,6 | 0,1 |
| kein Enzym | 0,2 | 0,0 |

Aus der Tabelle ist ersichtlich, daß die Weiße von Proben mit Manganoxidasen und MnSO₄ im Vergleich zu Proben ohne Enzym und ohne MnSO₄ um zwischen 1,6 und 2,7 Punkte erhöht war.

### Beispiel 8:

### Organische Synthese

Die im folgenden gezeigten Beispiele demonstrieren, daß ein erfindungsgemäßes Verfahren zur gezielten Synthese organischer Substanzen geeignet ist.
a) Oxidation von Alkoholgruppen zu Aldehyden
   22 ml einer 30 mM Malonatlösung pH 7,5 mit 0,5 mM MnSO₄ wurden bei 45°C mit 269 mg (1,6 mmol) 3,4-Dimethoxybenzylalkohol in 1 ml Ethanol versetzt. Nach 10 min Inkubation wurde die Manganoxidase (40 U) zugegeben. Nach 24 h Reaktionszeit wurde die Reaktionslösung mit Chloroform extrahiert und NMRspektroskopisch untersucht. Im Falle der Manganoxidase aus Leptothrix betrug die Ausbeute 32 % 3,4-Dimethoxybenzaldehyd, im Falle der Manganoxidase aus Bacillus betrug die Ausbeute 27 % 3,4-Dimethoxybenzaldehyd.
b) Oxidation von Alkoholen zu Ketonen
   22 ml einer 30 mM Malonatlösung pH 7,5 mit 0,5 mM MnSO₄ wurden bei 45°C mit 196 mg (1,6 mmol) 1-Phenylethanol versetzt. Nach 10 min Inkubation wurde die Manganoxidase (40 U) zugegeben. Nach 24 h Reaktionszeit wurde die Reaktionslösung mit HPLC untersucht. Im Falle der Manganoxidase aus Leptothrix betrug die Ausbeute 17 % Acetophenon, im Falle der Manganoxidase aus Bacillus betrug die Ausbeute 19 % Acetophenon.
   In beiden Beispielen konnte das erfindungsgemäße Oxidationsverfahren erfolgreich zur Synthese definierter Substanzen verwendet werden. Die verwendeten Substrate sollen hier nur exemplarisch das Oxidationsvermögen des Verfahrens demonstrieren und den Bereich der synthetisierbaren Produkte nicht einschränken.

### Beispiel 9:

### Jeansbleiche

Quadratisch zugeschnittene Stücke gefärbten Jeansstoffs (9 g /160 cm²) wurden in einem geschlossenen 500 ml Becher in einem Gesamtflüssigkeitsvolumen von 11,5 ml bei 45°C zusammen mit Manganoxidase, 0,2 mM MnSO₄ und 15 mM Komplexbildner (Oxalat) inkubiert. Der pH-Wert der Ansätze betrug 7,0 (15 mM Oxalat). Pro Gramm Stoff wurden 10 U Manganoxidase zugegeben. Nach 4 Stunden Inkubation wurden die Stoffstücke unter fließendem Wasser ausgewaschen, bis das Waschwasser farblos war. Die Stoffstücke wurden in einem Blattrockner getrocknet, anschließend gepreßt und mit einem geeigneten Spektralphotometer optisch bewertet.

Der Grad der Ausbleichung wurde mit einem Spektralphotometer CM 3700d (Minolta) entsprechend den Herstellerangaben bestimmt. Gemessen wurde ohne Glanz und ohne UV. Die Helligkeit der Proben wurde als prozentualer Wert der Totalreflektion im Vergleich zu einem Weissestandard (R 457) ermittelt. L* ist das Maß für die Helligkeit (weiß = 100; schwarz = 0).

Die Werte der behandelten Stoffe wurde mit den Werten unbehandelter Referenzstoffe verglichen. Die Veränderung der Helligkeit (ΔL*) der Proben im Vergleich zu unbehandelten Kontrollen wurde mit der Software PP2000 (Opticontrol) berechnet.

| | ΔL* |
|---|---|
| Kontrolle | 0 |
| Leptothrix | 23,14 |
| Bacillus | 28,72 |

Eine Veränderung des Helligkeitswertes (ΔL*) von 5 ist bereits mit dem Auge sichtbar, d.h. mit beiden Manganoxidasen konnte eine signifikante Bleichwirkung erreicht werden.

## Patentansprüche

1. Mehrkomponentensystem zur mediatorabhängigen enzymatischen Oxidation umfassend einen Oxidationskatalysator, ein Oxidationsmittel, und einen Mediator, **dadurch gekennzeichnet, daß**
a) der Oxidationskatalysator ausgewählt ist aus der Gruppe der Manganoxidasen,
b) das Oxidationsmittel ausgewählt ist aus der Gruppe Sauerstoff und sauerstoffhaltige Verbindungen,
c) der Mediator ausgewählt ist aus der Gruppe der Verbindungen, die Mn-Ionen enthalten.

2. Mehrkomponentensystem gemäß Anspruch 1, **dadurch gekennzeichnet, daß** es zusätzlich einen Komplexbildner umfaßt, ausgewählt aus der Gruppe der Komplexbildner, die Mn-Ionen komplexieren können.

3. Mehrkomponentensystem gemäß Anspruch 1 oder 2, **dadurch gekennzeichnet, daß** als Mediator Mn²⁺ oder Mn³⁺ Ionen dienen.

4. Mehrkomponentensystem gemäß Anspruch 2 oder 3, **dadurch gekennzeichnet, daß** der Komplexbildner keinen Stickstoff enthält, leicht bioabbaubar und toxikologisch unbedenklich ist.

5. Mehrkomponentensystem gemäß einem der Ansprüche 1 bis 4, **dadurch gekennzeichnet, daß** der Sauerstoff entweder direkt gasförmig oder in Form von flüssigem Sauerstoff oder als sauerstoffhaltiges Gasgemisch vorhanden ist.

6. Verfahren zur Oxidation eines Substrats **dadurch gekennzeichnet, daß** eine Manganoxidase in Anwesenheit von Sauerstoff und ggf. einem Komplexbildner für Mn-Ionen durch direkte Mn²⁺ Oxidation Mn³⁺ bildet und daß das Mn³⁺ Ion das Substrat oxidiert, dabei selbst zu Mn²⁺ reduziert wird und wiederum zur direkten Oxidation durch die Manganoxidase zur Verfügung steht.

7. Verfahren gemäß Anspruch 6 **dadurch gekennzeichnet, daß** das Substrat in Form einer wässrigen Lösung, Mischung oder Suspension eingesetzt wird.

8. Verfahren gemäß Anspruch 6 oder 7 **dadurch gekennzeichnet, daß** Manganionen in Konzentrationen zwischen 0,005 mM und 50 mM verwendet werden.

9. Verfahren gemäß Anspruch 6, 7 oder 8 **dadurch gekennzeichnet, daß** Sauerstoff mit einem Partialdruck von 0,05 - 5 bar eingesetzt wird.

10. Verfahren gemäß einem der Ansprüche 6 bis 9, **dadurch gekennzeichnet, daß** die Komplexbildner in Konzentrationen zwischen 1 mM und 500 mM verwendet werden.

## Claims

1. Multicomponent system for mediator-dependent enzymatic oxidation comprising an oxidation catalyst, an oxidizing agent, and a mediator, **characterized in that**
a) the oxidation catalyst is selected from the group of manganese oxidases,
b) the oxidizing agent is selected from the group consisting of oxygen and oxygen compounds,
c) the mediator is selected from the group of compounds which contain Mn ions.

2. Multicomponent system according to Claim 1, **characterized in that** it additionally comprises a complexing agent selected from the group of complexing agents that can complex Mn ions.

3. Multicomponent system according to Claim 1 or 2, **characterized in that** the mediator is Mn²⁺ or Mn³⁺ ions.

4. Multicomponent system according to Claim 2 or 3, **characterized in that** the complexing agent does not contain nitrogen, is readily biodegradable and is toxicologically harmless.

5. Multicomponent system according to one of Claims 1 to 4, **characterized in that** the oxygen is present either directly in the gaseous form or in the form of liquid oxygen or as oxygen-containing gas mixture.

6. Process for oxidizing a substrate, **characterized in that** a manganese oxidase, in the presence of oxygen and, if appropriate, a complexing agent for Mn ions, forms Mn³⁺ by direct Mn²⁺ oxidation, and **in that** the Mn³⁺ ion oxidizes the substrate, itself being reduced to Mn²⁺ and, in turn, being available for direct oxidation by the manganese oxidase.

7. Process according to Claim 6, **characterized in that** the substrate is used in the form of an aqueous solution, mixture or suspension.

8. Process according to Claim 6 or 7, **characterized in that** manganese ions are used at concentrations between 0.005 mM and 50 mM.

9. Process according to Claim 6, 7 or 8, **characterized in that** oxygen having a partial pressure of 0.05 - 5 bar is used.

10. Process according to one of Claims 6 to 9, **characterized in that** the complexing agents are used at concentrations between 1 mM and 500 mM.

## Revendications

1. Système multicomposant destiné à l'oxydation enzymatique médiateur-dépendante, comprenant un catalyseur d'oxydation, un oxydant et un médiateur, **caractérisé en ce que**
a) le catalyseur d'oxydation est choisi parmi le groupe constitué des oxydases de manganèse,
b) l'oxydant est choisi parmi le groupe constitué de l'oxygène et de composés oxygénés,
c) le médiateur est choisi parmi le groupe constitué des composés renfermant des ions Mn.

2. Système multicomposant selon la revendication 1, **caractérisé en ce qu'**il comprend en outre un agent complexant, choisi parmi le groupe constitué des agents complexants qui complexent des ions Mn.

3. Système multicomposant selon la revendication 1 ou 2, **caractérisé en ce que** des ions Mn²⁺ ou Mn³⁺ servent de médiateur.

4. Système multicomposant selon la revendication 2 ou 3, **caractérisé en ce que** l'agent complexant ne renferme pas d'azote, est aisément biodégradable et sans danger du point de vue toxicologique.

5. Système multicomposant selon l'une quelconque des revendications 1 à 4, **caractérisé en ce que** l'oxygène est présent soit directement sous forme gazeuse ou sous forme d'oxygène liquide, soit sous forme d'un mélange gazeux contenant de l'oxygène.

6. Procédé d'oxydation d'un substrat, **caractérisé en ce qu'**une oxydase de manganèse, en présence d'oxygène et éventuellement d'un agent complexant pour les ions Mn, forme Mn³⁺ par oxydation directe de Mn²⁺, et **en ce que** l'ion Mn³⁺ oxyde le substrat, étant lui-même réduit en Mn²⁺ et étant de nouveau disponible pour l'oxydation directe par l'oxydase de manganèse.

7. Procédé selon la revendication 6, **caractérisé en ce que** le substrat est utilisé sous forme d'une solution, d'un mélange ou d'une suspension aqueux.

8. Procédé selon la revendication 6 ou 7, **caractérisé en ce que** les ions manganèse sont utilisés en concentrations comprises entre 0,005 mM et 50 mM.

9. Procédé selon la revendication 6, 7 ou 8, **caractérisé en ce que** l'oxygène est utilisé avec une pression partielle de 0,05 à 5 bars.

10. Procédé selon l'une quelconque des revendications 6 à 9, **caractérisé en ce que** les agents complexants sont utilisés en concentrations comprises entre 1 mM et 500 mM.
